# EUROPÄISCHE PATENTANMELDUNG

(11) **EP 1 953 284 A2**
(43) Veröffentlichungstag der Anmeldung: **06.08.2008**
(21) Anmeldenummer: 07021101.6
(22) Anmeldetag: 29.10.2007
(51) Int. Cl.: D04B 1/18

(54) **Medizinisches Gestrick**

(30) Priorität: 05.02.2007 DE 102007006453
(71) Anmelder: OFA Bamberg GmbH, 96052 Bamberg (DE)
(72) Erfinder: Gebuhr, Helmut, 96047 Bamberg (DE)
(74) Vertreter: Gerber, Wolfram

(57) **Zusammenfassung**

Die Erfindung betrifft ein medizinisches elastisches Gestrick, zumindest einen eingelegten elastischen Faden zur Erzeugung einer Kompressionskraft aufweisend, wobei mindestens ein Teilbereich des Gestricks, in dem der elastische Faden ebenfalls eingelegt ist, mit einer größeren Maschengröße bzw. -weite gestrickt ist als der überwiegende Teil des Gestricks.

## Beschreibung

Die vorliegende Erfindung betrifft ein medizinisches elastisches Gestrick, zumindest einen eingelegten elastischen Faden zur Erzeugung einer Kompressionskraft aufweisend.

Medizinische Gestricke sind allgemein bekannt. Sie werden für Bandagen Strümpfe oder Strumpfhose, sowie Handschuhe, etc. eingesetzt. Sofern die medizinischen Gestricke eine Kompressionskraft ausüben sollen, wird ein elastischer Faden in das Gestrick eingelegt, welcher die Kompressionskraft erzeugt. Die Gestricke werden mittels Flach- und/oder Rundstrickverfahren hergestellt. Nachteilig bei diesen medizinischen Gestricken ist, dass die Kompressionskraft überall annähernd konstant über die gesamte Anlagefläche des Gestricks auf den Körper wirkt. Dies kann gerade im Gelenkbereich dazu führen, dass hohe Druckkräfte bei Bewegung auftreten. So kann es z.B. bei einer Kniebandage zum Einschneiden der Kniekehle und erhöhten Druck auf die Patellasehne beim Anwinkeln des Beines kommen.

Gleichsam ergibt sich oft ein schlechter Tragekomfort durch die Verwendung der bislang verwendeten medizinischen Gestricke.

Bei den bislang bekannten Flachstrickverfahren mit eingelegtem elastischen Schuss wurde bisher die erhöhte Dehnung bzw. ein besserer Tragekomfort durch Zugabe von Maschen erreicht. Das heißt, die Maschenzahl pro Strickreihe wird erhöht, um eine entsprechend niedrigere Kompression für bestimmte Bereiche zu erzielen.

Es ist zudem bekannt, im Leibteil von medizinischen Hosen, welche keinen eingelegten elastischen Faden und somit keine Kompression aufweisen, das Gestrick im Po-Teil lockerer herzustellen, da hier mehr Umfang benötigt wird um eine vorteilhafte Passform zu erzielen. Dies wird nur im Zusammenhang mit einem sogenannten Plus-/Minusleibteil (Gestrick im Poteil: 1:1 Glatt, Vorderteil: Micro-Mesh) verwendet.

Der Erfindung liegt daher die Aufgabe zugrunde, ein medizinisches Gestrick bereitzustellen, welches einen erhöhten Tragekomfort bietet.

Diese Aufgabe wird erfindungsgemäß dadurch gelöst, dass das Gestrick zumindest einen Teilbereich aufweist, in dem die Maschengröße gegenüber dem übrigen, den Kompressionsdruck erzeugenden Bereich bzw. Bereichen größer ausgebildet ist. Dieser zumindest eine Teilbereich erzeugt einen geringeren Kompressionsdruck, da sie stärker dehnbar sind. Die Erfindung schließt nicht aus, dass die Teilbereiche selbst ebenfalls einen Kompressionsdruck aufbauen, insbesondere durch den eingelegten elastischen Faden.

Bestimmten Körpersegmenten können der bzw. die Teilbereich(e) mit größerer Maschengröße zugeordnet werden. Dies kann sich auf das gesamte Körpersegment oder auf Teile des Körpersegmentes beziehen. Vorteilhafterweise sollte(n) der bzw. die Teilbereich(e) in der Form und Größe so ausgelegt werden, dass der gewünschte Körperbereich überdeckt wird, bzw. die entsprechende Verdehnung möglich ist. Das medizinische Gestrick kann dabei sowohl im Rund- als auch im Flachstrickverfahren hergestellt werden.

Die Maschengröße kann sich beim erfindungsgemäßen Gestrick auch innerhalb einer Maschenzeile verändern. Hierdurch ist es möglich, dass die Gestalt der Teilbereiche mit größerer Maschengröße beliebig ausgebildet sein kann.

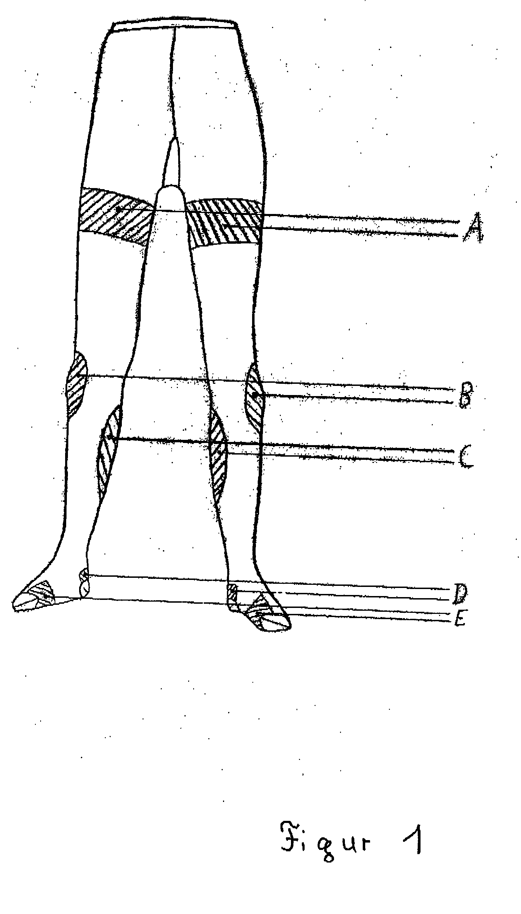

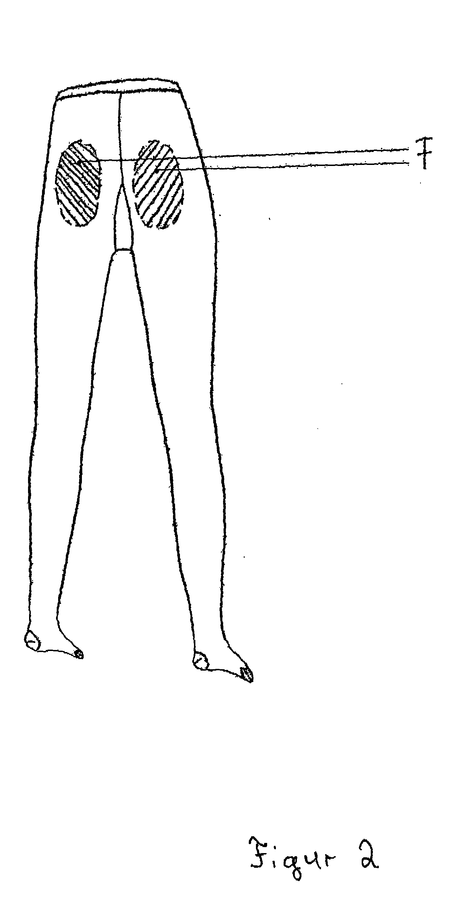

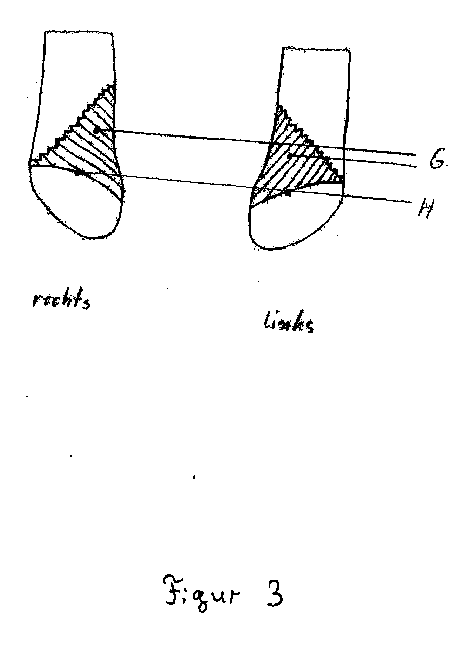

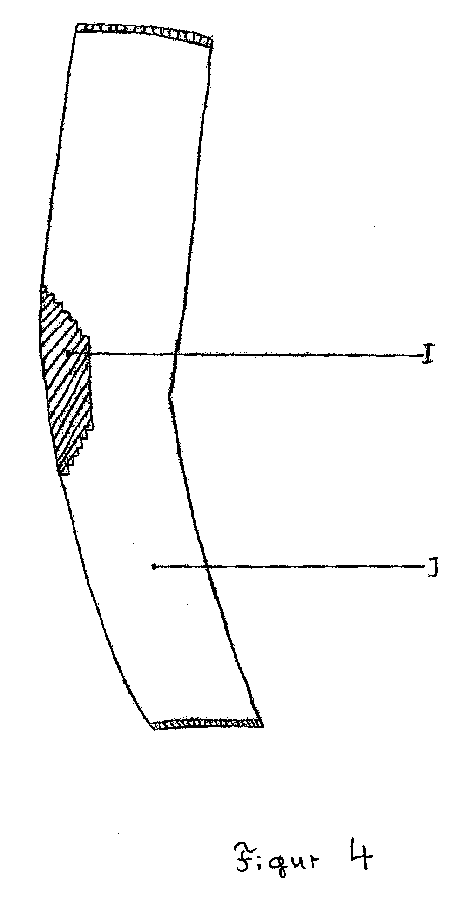

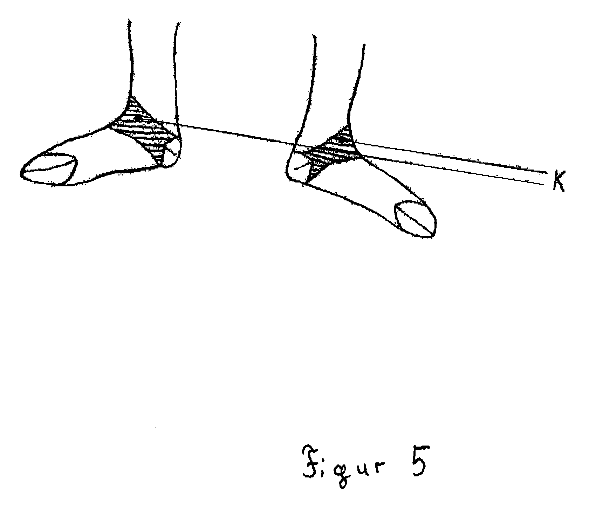

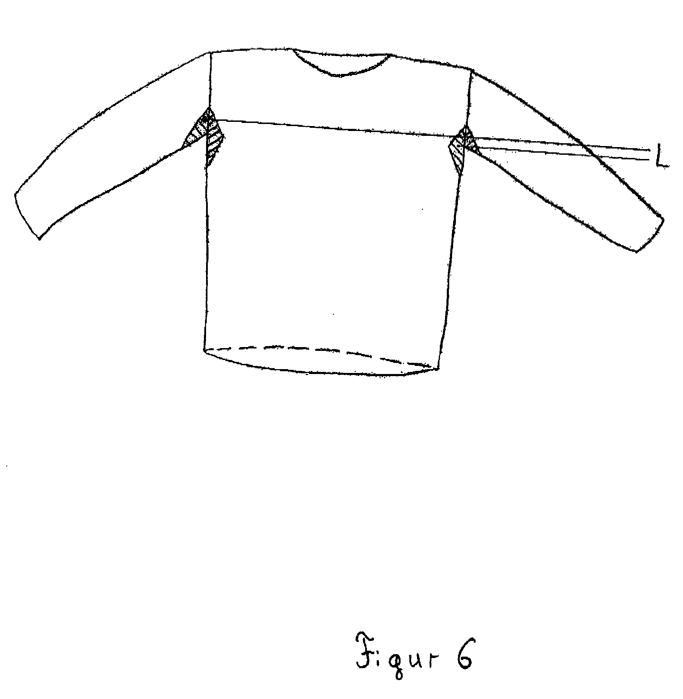

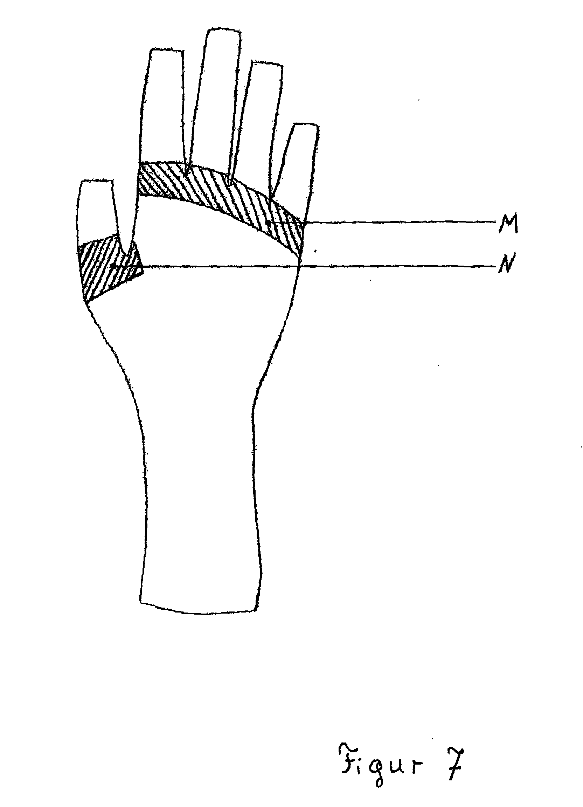

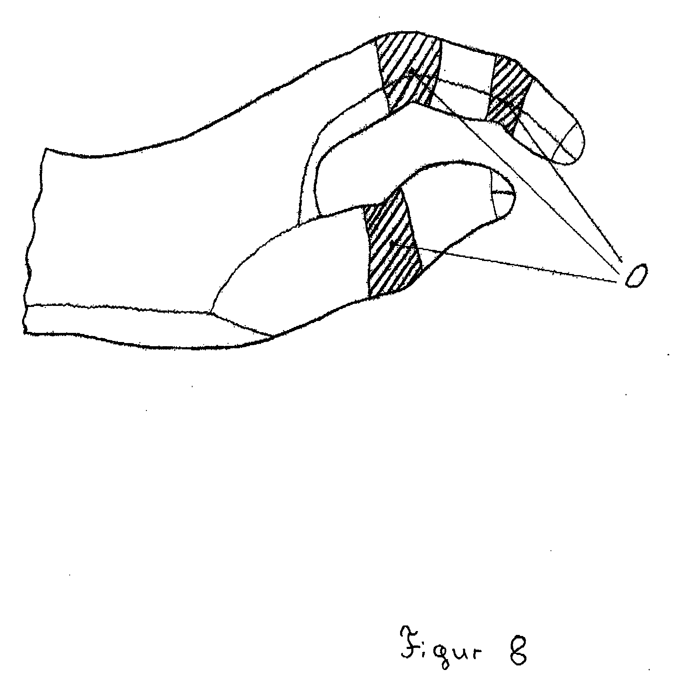

Nachfolgend wird nur eine bevorzugte Auswahl der Köpersegmente aufgeführt, für die das erfindungsgemäße Gestrick einen erhöhten Tragekomfort bietet:
1. Gestricke, die im Rund- oder Flachstrickverfahren hergestellt werden:
   a) Im Fußbereich kann die vordere innere Hälfte des Fußes, insbesondere zwischen Ferse und Zehenbereich oder im Mittelfußknochenbereich und/oder Bereich des Großzehengrundgelenks, mit größerer Masche gestrickt werden. Dadurch wird erreicht, dass sich das Gestrick in diesem Bereich leichter dehnen lässt, bzw. länger gezogen werden kann. Der annähernd dreiecksförmige Kompressionsteil passt sich hierbei besser dem anatomischen Zehenverlauf an. Die Figur 1 (Bezugzeichen E) sowie Figur 3 (Bezugszeichen G) zeigen die Bereiche des Fußes, in denen die Maschengröße gegenüber dem übrigen Gestrick vergrößert werden kann. Die Teilbereiche mit der größeren Maschenweite ist, wie in den übrigen Figuren auch, schraffiert dargestellt.
      Wie in den Figuren 1 und 3 dargestellt, kann die Einarbeitung des Teilbereichs mit größerer Maschengröße entweder gerade, d.h. von einer Maschenzeile zur nächsten erfolgt eine Änderung der Maschengröße, oder aber auch schräg erfolgen (Teilbereich G). Der Übergang des Kompressionsteils in die angestrickte Fußspitze (H) kann vorteilhaft schräg verlaufend ausgebildet werden, um dem Zehenansatz zu folgen. Hierdurch wird vorteilhaft erreicht, dass die Zehen nicht unnötig komprimiert werden.
   b) Damit der Druck auf die Achillessehne verringert wird, kann in diesem Bereich die Maschengröße größer gestellt werden. Der hierfür erforderliche Bereich ist in Figur 1 mit dem Bezugszeichen D gekennzeichnet. Das Gestrick wird im Bereich der Achillessehne lockerer, d.h. mit größerer Maschengröße, gestrickt, um einen verbesserten Tragekomfort beim Laufen, durch die Überdehnung längs der Sehne, zu erzielen.
   c) Denkbar ist auch, den Bereich C (Figur 1) der Wade elastischer zu gestalten, um hier die phlebologischen Eigenschaften zu verbessern - niedriger Stiffness.
   d) Im Bereich B (Figur 1) der Kniescheibe können die Maschen ebenfalls lockerer gestrickt werden, so dass beim Anwinkeln des Knies der Druck auf die Patella abnimmt bzw. ebenso das Einschneiden der Kniekehle verringert wird.
   e) Um die unterschiedlichen Längen des Vorder- und Rückbeins auszugleichen, kann z.B. die Maschengröße vorne im Oberschenkelbereich A im Vergleich zur Oberschenkelrückseite größer ausgebildet sein, so dass sich der Kompressionsteil besser dem Beinübergang zur Hüfte anpasst. Diese Ausbildung ist ebenso bei einem Halbschenkelstrumpf oder Wadenstrumpf möglich.
   f) Beim Leibteil mit voller Kompression kann der Poteil vorteilhaft mit größerer Masche gestrickt werden, so dass der entsprechende Gestrickteil F, Figur 2, hinten länger und breiter ausfällt. Dies wirkt sich vorteilhaft aus, bei der Auswahl des Hosenzwickels. Er kann somit entsprechend klein gewählt werden. Ebenso ist denkbar, nur im Po-Bereich lockerer zu stricken, um einen gewissen Po-up Effekt (Anheben des Po's) zu erzielen (Gestrickbindung: Rechts/Links 1:1).
   g) Bei Armstrümpfen kann der Ellenbogenbereich I, Figur 4, außenseitig lockerer gestrickt werden. Beim Anwinkeln des Armes wird somit ein Einschneiden des Kompressionsteiles in der Armbeuge vorteilhaft verhindert bzw. vermindert.
   h) Das Vorsehen des erfindungsgemäßen Teilbereichs mit größerer Maschengröße ist auch im Fersenbereich bzw. am Rist K, Figur 5, möglich, um das Einschneiden über das Ristmaß zu vermeiden bzw. zu vermindern, in dem beim Laufen der Druck auf das untere Halteband der Streckermuskulatur kleiner ist.
2. Gestricke, die ausschließlich im Flachstrickverfahren hergestellt werden:
   a) Bei Thoraxbandagen kann der Bereich L, Figur 6, unter dem Armansatz an der Schulter mit größeren Maschen gestrickt bzw. ausgebildet sein, damit in diesem Bereich der Kompressionsdruck abnimmt. Hierdurch wird eine bessere Bewegungsfreiheit beim Anheben des Armes zu erreicht bzw. ein besseres Anschmiegen des Gestrickes an die Haut möglich.
   b) Ebenso ist es möglich, bei Handschuhen mit Kompressionsteil den Tragekomfort zu verbessern in dem am Handrücken in den Bereichen M und N (Figur 7) der vier Fingeransätze sowie des Daumenansatzes die Maschen vorteilhaft größer gestellt werden. Beim Krümmen der Finger kann das Gestrick am Handrücken vorteilhaft gedehnt werden, so dass ein Einschneiden auf der Handinnenseite verringert wird. Zusätzlich könnte im Gelenkbereich 0, Figur 8, der Fingerknöchel das Gestrick auf der Oberseite lockerer gestrickt werden, so dass beim Anwinkeln ein Einschneiden der Finger verringert und die Blutzirkulation nicht behindert wird. Hier werden die phlebologischen Eigenschaften verbessert - niedriger Stiffness.
3. Gestricke, die ausschließlich im Rundstrickverfahren hergestellt werden:
   Fußteile von Strümpfen werden ausschließlich im Rundstrickverfahren hergestellt. Die Fußspitze wird entweder angenäht oder lediglich zugenäht. In diesem Zusammenhang wird die Konstruktion der geschlossenen Spitze (Fischmaulspitze) näher erläutert, welche den Druck auf die Zehen bzw. den Tragekomfort erhöht. Dies gilt insbesondere für medizinische Strümpfe mit Kompression bzw. mit eingelegtem gummielastischen Faden. Hierbei wird in der gummielastischen Spitze ein mindestens 78 dtex Elastanfaden mit kreuzweiser Umwindung verwendet. Die Gesamtfadenstärke wird so dick gewählt, um zum einen die Haltbarkeit in der Spitze zu gewährleisten, und zum anderen der Gestrickdicke des Strumpfes zu entsprechen. Der Nachteil ist konstruktionsbedingt. Es entsteht ein unangenehmer Druck auf die Zehen, d.h. die Zehen werden zusammengedrückt. Um diesen Nachteil auszugleichen wurde bisher der elastische Kernfaden Elastan 78 dtex durch einen vorzugsweise dünneren 44 dtex Faden ersetzt. Die Umwindung wurde entsprechend dicker gewählt, so dass die Gesamtfadenstärke mit einem 78 dtex Elastanfaden vergleichbar ist. Der Vorteil liegt in der höheren Dehnung und bewirkt somit ein angenehmen Tragekomfort für die Spitze. Bisher wird diese Spitze aber nicht angestrickt, sondern nur am Fußteil angenäht. Der Nachteil ist die wulstige Naht und unerwünschte Druckstellen.
   Durch das vorteilhaft erfindungsgemäße lockere Stricken von Teilbereichen, kann nunmehr die Spitze vorteilhaft angestrickt, und eine Naht vermieden werden. Eine weitere Verbesserung ergibt sich, sofern auch der Kompressionsfußteil innenseitig lockerer gestrickt wird (siehe auch unter Rund- und Flachstrickverfahren, Punkt 1a).
   Selbstverständlich ist die erfindungsgemäße Maschenverstellung auch für andere Bereiche bzw. Kompressionssegmente denkbar. Ebenso, und dies gilt für alle Ausführungsformen gemäß dieser Erfindung, muss der Übergang von der normalen Maschengröße zur vergrößerten Maschengröße nicht sprunghaft von einer Masche bzw. Maschenzeile zur nächsten erfolgen, sondern kann auch fließend erfolgen, d.h. über mehrere benachbarte Maschen und/oder Maschenzeilen erfolgt eine Größenänderung der Maschengröße.

Das Erzeugen von größeren Maschengrößen erfolgt beim Rundstricken wie folgt:
Die Maschengröße wird durch den Hub der Stricknadel zur Platine in der Abschlagsstellung bestimmt. Der Nadelhub wird folgendermaßen gesteuert:
   Der Abstand des Zylinder sowie die Platinenstellung zur Nadel bleiben gleich, die Nadel wird durch die Strickschlösser ausgetrieben. Die Strickschlösser können je nach Grundstellung um einen bestimmten Hub verändert, d.h. angehoben bzw. abgesenkt, werden. Die Strickschlösser werden dabei vom Strickprogramm gesteuert. Beim Maschenbildungsprozess wird die Maschengröße von einer Masche zur Folgenden verändert. Dadurch lassen sich Bereiche mit verschiedenen Profilen einstricken, welche eine größere Maschengröße im Vergleich zu angrenzenden Maschen außerhalb dieser Bereiche aufweisen.
   Das Erzeugen von größeren Maschengrößen erfolgt beim Flachstricken wie folgt:
      Die Maschengröße wird durch den Hub der Stricknadel zur Platine bestimmt. Der Nadelhub wird folgendermaßen gesteuert:
         Die Platinenstellung bleibt gleich, die Nadel wird durch die Strickschlösser unterschiedlich ausgetrieben, welche stufenlos angesteuert werden können.

## Patentansprüche

1. Medizinisches elastisches Gestrick, zumindest einen eingelegten elastischen Faden zur Erzeugung einer Kompressionskraft aufweisend, **dadurch gekennzeichnet, dass** mindestens ein Teilbereich des Gestricks, in dem der elastische Faden ebenfalls eingelegt ist, mit einer größeren Maschengröße bzw. -weite gestrickt ist als der überwiegende Teil des Gestricks.

2. Gestrick nach Anspruch 1, **dadurch gekennzeichnet, dass** das Gestrick ein Kompressionsstrumpf, eine Kompressionsstrumpfhose oder eine Bandage ist.

3. Gestrick nach einem der vorhergehenden Ansprüche, **dadurch gekennzeichnet, dass** die Teilbereiche mit einer größeren Maschengröße eine geringere Kompression aufweisen als der überwiegende Teil des Gestricks.

4. Gestrick nach einem der vorhergehenden Ansprüche, **dadurch gekennzeichnet, dass** ein Teilbereich im Bereich der Kniescheibe angeordnet ist, derart, dass das Gestrick beim Beugen des Knies die Patella weniger stark druckbeaufschlagt.

5. Gestrick nach einem der vorhergehenden Ansprüche, **dadurch gekennzeichnet, dass** im Bereich der die Zehen und/oder Achillessehne ein Teilbereich mit größerer Maschengröße angeordnet ist.

6. Gestrick nach einem der vorhergehenden Ansprüche, **dadurch gekennzeichnet, dass** im Bereich der Wade jeweils mindestens ein Teilbereich mit größerer Maschengröße angeordnet ist.

7. Gestrick nach einem der vorhergehenden Ansprüche, **dadurch gekennzeichnet, dass** ein Teilbereich sich im Bereich der Sitzfläche bzw. des Pos befindet.

8. Gestrick nach einem der vorhergehenden Ansprüche, **dadurch gekennzeichnet, dass** das Gestrick ein Strumpf- oder eine Strumpfhose ist, die zwischen dem Fußteil und dem Zehenteil einen insbesondere annähernd dreiecksförmig gestrickten Teilbereich mit größerer Maschengröße angeordnet ist.

9. Gestrick nach einem der vorhergehenden Ansprüche, **dadurch gekennzeichnet, dass** das Gestrick ein Strumpf- oder eine Strumpfhose ist, die im Ristbereich, dass heißt auf dem Fuß im Übergangsbereich von Fuß zum Bein, einen Teilbereich mit größerer Maschengröße aufweist.

10. Gestrick nach einem der Ansprüche 1 bis 3, **dadurch gekennzeichnet, dass** das Gestrick eine Thoraxbandage ist, die in den Armbeugen jeweils mindestens einen Teilbereich mit größerer Maschengröße aufweist.

11. Gestrick nach einem der Ansprüche 1 bis 3, **dadurch gekennzeichnet, dass** das Gestrick eine Handschuh ist, der im Übergangsbereich von der Hand zu den Fingern am Handrücken jeweils mindestens ein Teilbereich für jeden Finger oder ein durchgehender Teilbereich für Zeigefinger, Mittelfinger, Ringfinger und kleinem Finger und einen zusätzlichen Teilbereich für den Daumen mit größerer Maschengröße aufweist.

12. Gestrick nach einem der Ansprüche 1 bis 3, **dadurch gekennzeichnet, dass** das Gestrick eine Handschuh ist, der im Bereich zumindest eines Fingergelenkes einen Teilbereich mit größerer Maschengröße aufweist, wobei sich der Teilbereich insbesondere um den gesamten Finger herum oder nur entlang des Handrückens bzw. an der Fingeroberseite erstreckt.

13. Gestrick nach einem der vorhergehenden Ansprüche, **dadurch gekennzeichnet, dass** das Gestrick im Rund- oder Flachstrickverfahren erstellt ist.
